# EUROPEAN PATENT SPECIFICATION

(11) **EP 1 768 669 B1**
(45) Date of publication and mention of the grant of the patent: **11.11.2009**
(21) Application number: 05772459.3
(22) Date of filing: 15.07.2005
(51) Int. Cl.: A61K 31/455, A61P 33/02

(54) **PHARMACEUTICAL COMPOSITIONS FOR THE TREATMENT OF LEISHMANIASIS**
PHARMAZEUTISCHE ZUSAMMENSETZUNGEN ZUR BEHANDLUNG VON LEISHMANIASIS
COMPOSITIONS PHARMACEUTIQUES POUR LE TRAITEMENT DE LA LEISHMANIOSE

(30) Priority: 19.07.2004 US 588802 P
(43) Date of publication of application: 04.04.2007
(73) Proprietor: Institut de Recherche pour le Développement ( IRD), 75480 Paris Cedex 10 (FR)
(72) Inventor: OUAISSI, Ali, F-34150 La Boissière (FR); SERENO, Denis, F-34500 Poussan (FR); VERGNES, Baptiste, F-23500 Felletin (FR)
(74) Representative: Grosset-Fournier, Chantal Catherine
(86) International application number: PCT/EP2005/007715
(87) International publication number: WO 2006/008082

(56) References cited:
- WO-A-00/78268
- US-B1- 6 194 203
- US-B1- 6 339 073
- US-B1- 6 437 217
- SHEMAROVA I V ET AL: "Antiprotozoan effect of picolinic acid and some of its structural analogs" KHIMIKO-FARMATSEVTICHESKII ZHURNAL, vol. 34, no. 7, July 2000 (2000-07), pages 13-15, XP009054788 ISSN: 0023-1134
- AL-MULLA HUMMADI Y M ET AL: "Leishmania major and Leishmania tropica: I the in vitro effects of an immunomodulator, S2-Complex" EXPERIMENTAL PARASITOLOGY, NEW YORK, NY, US, vol. 111, no. 1, September 2005 (2005-09), pages 47-54, XP004995911 ISSN: 0014-4894
- SERENO D ET AL: "In vitro antileishmanial activity of nicotinamide" ANTIMICROBIAL AGENTS AND CHEMOTHERAPY, vol. 49, no. 2, February 2005 (2005-02), pages 808-812, XP009046614 ISSN: 0066-4804

## Description

Protozoans belonging to the *Tryparrosomatidae* family account for numerous pathologies afflicting man or animals.

Thus, among protozoans of the *Trypanosoma* genus, *T. brucei* and *T. cruzi* are for instance the etiological agents of sleep disease and Chagas disease.

Protozoans of the *Leishmania* genus, such as *L. aethiopica, L. donovani, L. infantum, L. major, L. mexicana* or *L. tropica* are responsible for leishmaniasis (also named leishmaniosis). Infections by these parasites are endemic in more than 88 countries. WHO estimates that more than 12 millions individuals are infected by these parasites and more than 350 millions would be exposed to infections daily. Three major forms of leishmaniosis are documented, among which the most dangerous form, visceral leishmaniosis, can have a lethal outcome in absence of treatment. This situation has worsened since the occurrence of HIV, because these infections are more frequently found as opportunistic infections in individuals afflicted by the acquired immunodeficiency syndrome (AIDS), in particular in South-West Europe. Parasites take advantage of the immunosuppressed status of the host to establish themselves or to reactivate.

Current leishmaniosis treatments are based on drugs difficult to handle, such as amphotericin B or drugs belonging to the antimonial family, which have serious side effects.

Niacin is the generic name for 2 compounds: nicotinamide (NAm) and nicotinic acid. Both were first used clinically in 1937, when these compounds were each shown to act as « pellagra-preventive » factor. High dose of NAm and its acid derivative nicotinic acid, are often used interchangeably to treat a number of conditions including anxiety, osteoarthritis, and psychosis. Furthermore, NAm is currently in trials as therapy to prevent cancer recurrence and insulin-dependent (Type I) diabetes (4). Beside this, activity of NAm has been evaluated in anti-*mycobacterium tuberculosis* studies performed during 1945-1961 and in anti-HIV studies performed from 1991 to the present (reviewed in 7).

Shemarova et al. (Khimiko-Farmatsevticheskii Zhurnal, vol. 324, n°.7, July 2000, pp13-15) discloses the antiparasitic efficacy of nicotinic acid.

Patent application WO 00/78268 discloses the use of nicotinamide in the treatment of malaria.

It is an object of the present invention to provide new medicaments, lacking the drawbacks of the currently used medicaments, for the treatment of protozoan parasitic diseases related to *Trypanosomatidae* family, such as leishmaniosis.

The present invention relates to the use of at least one compound of the following general formula (I): 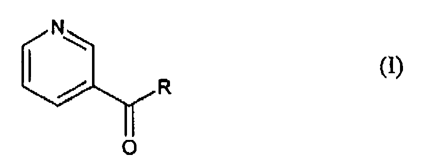 wherein R represents OH or NH₂,
or of the pharmaceutically acceptable salts of said compound
for the manufacture of a medicament intended for the prevention or the treatment of parasitic diseases related to *Trypanosomatidae* family, in particular *Trypanosoma* genus or *Leishmania* genus under their respective intracellular or extracellular forms, more particularly of leishmaniosis, and especially for the prevention or the treatment of parasitic diseases related to *Trypanosomatidae* family occurring in immunodepressed patients.

The expression "parasites" relates to unicellular eukaryotic organisms which are able to infect mammals and to survive and/or multiply in the infected mammal.

As intended herein "parasitic diseases" relate to diseases caused by parasites as defined above.

Advantageously, the use of compounds of formula (I) for the prevention or the treatment of parasitic diseases is sound, since numerous bioavailability studies have assessed that high plasma concentrations of these compounds, e.g. 2,3 mM, could be achieved without serious side effects.

In a preferred embodiment of the above defined use of a compound of formula (I), R represents OH, said compound corresponding to niacin (vitamin B3), of the following formula (II):

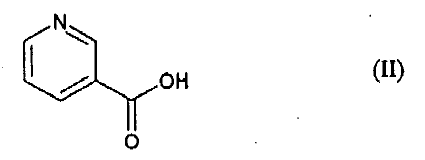

In another preferred embodiment of the above defined use of a compound of formula (I), R represents NH₂, said compound corresponding to nicotinamide, of the following formula (III):

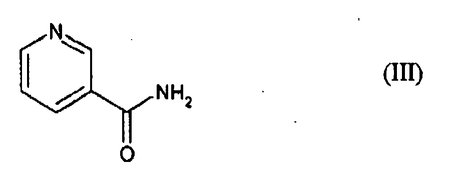

According to another preferred embodiment of the above defined use, the medicament is suitable for an administration of the compound of formula (I) by oral, intravenous, topical or intralesional route.

As intended herein "intralesional" means that the medicament is suitable to be administered at the sites of parasite-caused skin lesions of patients, in particular in case of *Leishmania* infections.

According to a particularly preferred embodiment of the above defined use, the medicament is suitable for an administration of the compound of formula (I) at a unit dose of about 10 mg to about 10 g, in particular of about 1 g to about 6 g.

According to another particularly preferred embodiment of the above defined use, the medicament is suitable for an administration of the compound of formula (I) at a dosage of about 5 mg/m²/day to about 5 g/m²/day, in particular of about 500 mg/m²/day to about 3 g/m²/day.

In another preferred embodiment of the above defined use, the compound of formula (I) in association with at least one anti-parasitic compound, such as a compound selected from:
miltefosin, antimonials, amphotericin B, benznidazol, nifurtimox, paromomycin, pentamidin and its derivatives, arsenic derivatives, melarsopol and difluoromethylornithin.

Advantageously, the association of a compound of formula (I) with an anti-parasitic compound has additive or synergic effects which enables a diminished administration of said anti-parasitic compound and thus diminished side effects.

The present invention also relates to a pharmaceutical composition comprising as active substances:
- at least one compound of the following general formula (I): 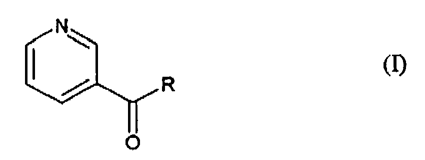 wherein R represents NH₂,
   or of the pharmaceutically acceptable salts of said compound, and
- antimonials,
- in association with a pharmaceutically acceptable carrier.

According to a preferred embodiment, the above defined pharmaceutical composition is suitable for an administration by oral intravenous, topical or intralesional route.

According to another preferred embodiment, the above defined pharmaceutical composition is suitable for the administration of the compound of formula (I) at a unit dose of about 10 mg to about 10 g, in particular of about 1 g to about 6 g.

According to yet another preferred embodiment, the above defined pharmaceutical composition is suitable for the administration of the compound of formula (I) at a dosage of about 5 mg/m²/day to about 5 g/m²/day, in particular of about 500 mg/m²/day to about 3 g/m²/day.

The present invention also relates to products containing:
- at least one compound of the following general formula (I): 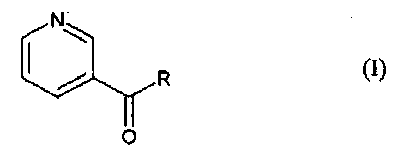 wherein R represents NH₂,
   or the pharmaceutically acceptable salts of said compound, in association with
- at least one anti-parasitic compound, such as a compound selected from: antimonials,
as a combined preparation for simultaneous, separate or sequential use in the prevention or the treatment of parasitic diseases, related to *Trypanosomatidae* family, in particular *Trypanosoma* genus or *Leishmania* genus under their respective intracellular or extracellular forms, more particularly of leishmaniosis, and especially for the prevention or the treatment of parasitic diseases occurring in immuno-depressed patients.

### DESCRIPTION OF THE FIGURES

### Figure 1A, Figure 1B, Figure 1C, and Figure 1D

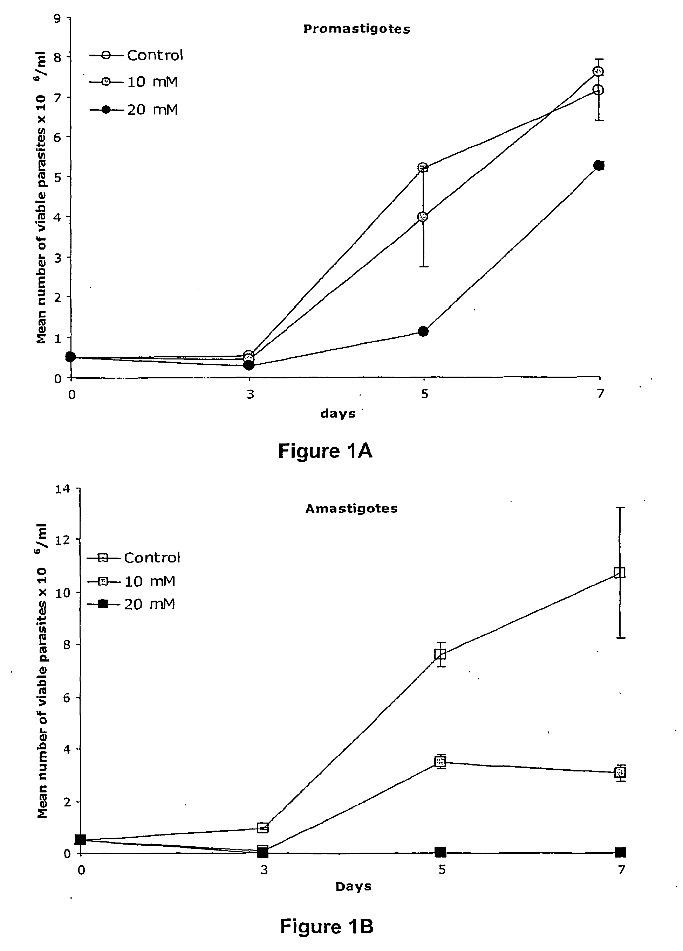

Figure 1A represents the mean number of viable leishmania at the promastigote stage (vertical axis, x10⁶ / ml) as a function of time (horizontal axis, days), in presence of no nicotinamide (NAm) (control, white circles), 10 mM NAm (grey circles), or 20 mM NAm (black circles).
   Figure 1B represents the mean number of viable axenically grown amastigotes leishmania (vertical axis, x10⁶ / ml) as a function of time (horizontal axis, days), in presence of no nicotinamide (NAm) (control, white squares), 10 mM NAm (grey squares), or 20 mM NAm (black squares).

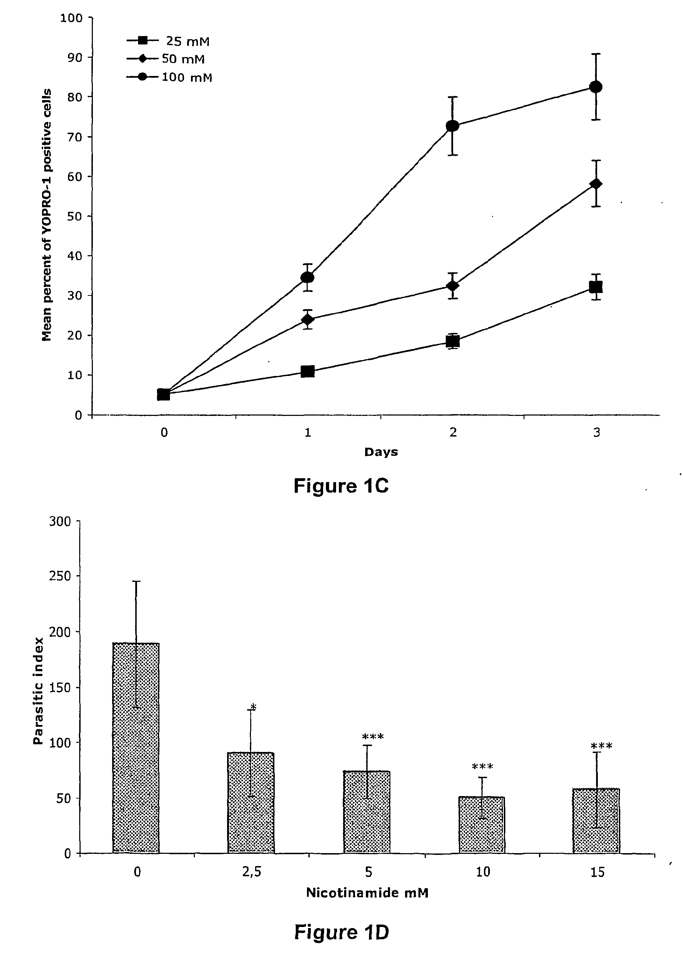

   Figure 1C represents the mean percentage of YOPRO-1-positive axenically grown amastigotes (i.e. apoptotic cells) (vertical axis) as a function of time (horizontal axis, days) in presence of 25 mM Nam (squares), 50 mM Nam (diamonds), or 100 mM Nam (circles). Results are expressed as a mean of a triplicate experiment.
   Figure 1D represents the parasitic index (vertical axis) as a function of NAm concentration (horizontal axis, mM). Results are representative of one over two experiments carried out in sextuplate (one star (*) corresponds to P<0.05, two stars (**) correspond to P<0.005, and three stars (***) correspond to P<0.001).
**Figure 2A and Figure 2B**

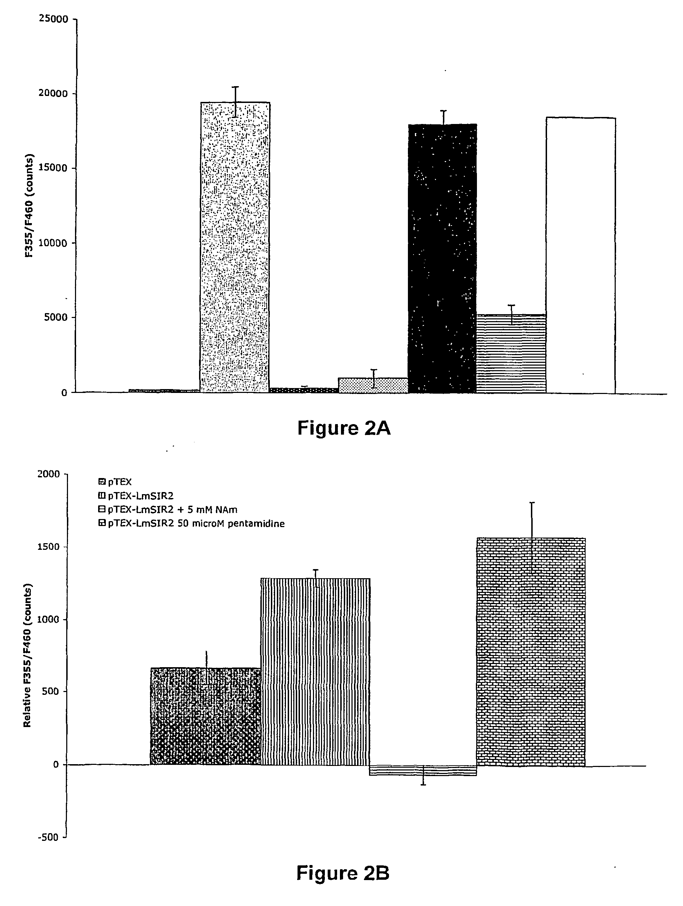

   Figure 2A represents the NAD-dependent deacetylase activity of the SIRT1 enzyme expressed as the fluorescence at 355 nm to fluorescence at 460 nm ratio (F355/F460) (vertical axis, counts) for (from left to right) a control assay without NAD (first histogram), a control assay with NAD (second histogram), an assay with 5 mM NAm (third histogram), an assay with 20 mM NAm (fourth histogram), an assay with 5 mM NAc (fifth histogram), an assay with 20 mM NAc (sixth histogram) and a control assay (seventh histogram).
   Figure 2B represents the NAD-dependent deacetylase activity detected in leishmania expressed as the relative fluorescence at 355 nm to fluorescence at 460 nm ratio (F355/F460) (vertical axis, counts) for leishmania carrying an empty pTEX plasmid (first histogram), leishmania carrying a plasmid expressing LmSIR2 (pTEX-LmSIR2) (second histogram), leishmania carrying a plasmid expressing LmSIR2 (pTEX-LmSIR2) in presence of 5 mM NAm and leishmania carrying a plasmid expressing LmSIR2 (pTEX-LmSIR2) in presence of 50 µM pentamidine. Results are given as a mean of two duplicate experiments.
**Figure 3A and Figure 3B**

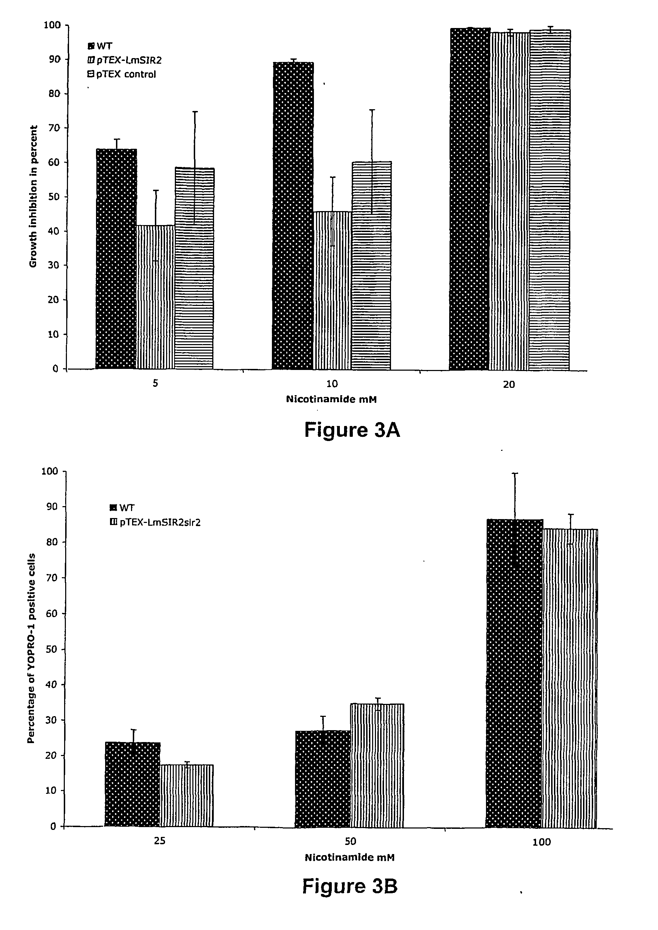

   Figure 3A represents the percentage of growth inhibition (vertical axis) as a function of NAm concentration (horizontal axis, mM) for wild type (WT) leishmania (black histogram), leishmania carrying a pTEX-LmSIR2 plasmid (vertically hatched histogram) or leishmania carrying a control pTEX plasmid (horizontally hatched histogram).
   Figure 3B represents the percentage of YOPRO-1 positive cells (vertical axis) as a function of NAm concentration (horizontal axis, mM) for wild type (WT) leishmania (black histogram), or leishmania carrying a pTEX-LmSIR2 plasmid (vertically hatched histogam).
Results are expressed as mean value of a quadruplate experiments.

### EXAMPLES

### Example 1

The growth of *Leishmania* amastigotes and promastigotes was followed in axenic culture conditions in the presence or absence of NAm.

A cloned line of *L. infantum* (MHOM/MA/67/ITMAP-263) was used in all experiments. Each subculture was initiated at 5 x 10⁵ parasites/ml of medium. Axenically grown amastigote forms of *L. infantum* were maintained at 37°C with 5% CO₂ by weekly subpassages in a cell-free medium called MAA/20 (medium for axenically grown amastigotes) in 25-ml flasks, as previously described (**10**). Promastigote forms were maintained at 26°C by weekly subpassage in SDM 79 medium supplemented with 10% foetal calf serum (FCS) and 100 units/ml penicillin and 100 □g/ml streptomycin. Nicotinamide (SIGMA, St Louis) was added at the appropriate concentration and the mean number of viable parasites determined using FACs analysis, as previously described (**11**).

As shown in **Figures 1A and 1B****,** NAm strongly inhibited the proliferation of both promastigotes and amastigotes with promastigote forms showing less sensitivity to NAm than amastigotes. At 20 mM NAm, the capacity of axenic amastigotes to proliferate was virtualy completely abrogated, whereas a delay in the growth of promastigotes occured. The growth inhibitory activity of nicotinamide was not restricted to *L. infantum* since *L. amazonsensis* amastigotes were also found to be sensitive to the activity of NAm. Furthermore, it was found that the acid derivative of NAm, the nicotinic acid (NicotAc or NAc), exerted a growth inhibitory activity towards *Leishmania* parasites, although at higher concentrations.

### Example 2

### The nature of NAm-induced amastigotes growth arrest was then investigated

Cells were seeded at 5 x 10⁵ parasites/ml and NAm was added at various concentrations ranging from 25 to 100 mM. After 24, 48 and 72 hours of incubation aliquots (10⁶ parasites) were collected, washed and incubated for 10 min with 10 □M of YOPRO-1, an apoptotic cell marker (Molecular probes). The mean percentage of YOPRO-1 positive cells was determined as previously described (**9**). At concentrations higher than 25 mM, NAm exerted a strong dose- dependent leishmanicidal activity against axenic amastigote, as demonstrated by the occurence of YOPRO-1 positive cells. Maximal effect was observed after 3 days of culture in the presence of 100 mM of NAm **(****Figure 1C****).**

Having observed that NAm induced axenic amastigotes death, it was of interest to examine its effect on intracellular amastigotes proliferation.

In a first series of experiments, THP-1 monocytes were incubated during 3 days with various concentrations of NAm and the growth and viability of cells were recorded. Up to 10 mM of NAm, no effect on cell growth and viability was observed. In contrast, 20 mM NAm inhibited the proliferation of THP-1 monocyte by about 45% in agreement with the values recorded for other cell types : SupT1 and PBLs cells (**6**).

Thus, THP-1 differentiated macrophages were infected with stationnary phase amastigotes at a host cell-parasite ratio of 5:1. After 4 hours, non adherent parasites were removed and nicotinamide was added to the medium at the appropriate concentration. After 3 days of incubation time, cells were fixed with methanol and stained with giemsa. Parasitic index PI (mean percentage of infected macrophages X number of amastigotes per macrophage) was determined. As shown in **Figure 1D****,** NAm significantly inhibited the *in vitro* proliferation of intracellular amastigote. Maximal activity was observed with 10 mM of NAm. At this concentration a reduction of almost 70% of PI was observed. Interestingly, at low dosage 2.5 mM NAm is also able to significantly inhibit intracellular amastigote proliferation when compared to control non treated cultures (p<0.05).

### Example 3

It has been recently demonstrated that NAm is a substrate of sir2-like enzymes *in vitro* (**5**). Therefore, complementary experiments were conducted in order to examine whether NAm could interfere with *Leishmania* deacetylase activity *in vitro.* To test this possibility, a commercially available "cyclex SIR2 assay kit" and SIRT1 as a standard enzyme (MBL, Japan) were used.

As shown in **Figure 2A****,** the deacetylase activity of SIRT1 is strictly dependent on the presence of NAD, addition of 5 mM or 20 mM NAm in the assay almost completely abrogated the enzymatic activity of SIRT1. In contrast, 5 mM of NicotAc had no significant effect, in agreement with the data reported by other investigators (2), whereas 20 mM of NicotAc showed a significant effect.

Having established a standard inhibitory assay, the effect of NAm was then examined on the NAD-dependent deacetylase activity contained in *Leishmania* extracts from mutant parasites carrying extra copies of LmSIR2 gene (pTEX-LmSIR2) or empty plasmid DNA (pTEX) (**11**).

Briefly, 2 10⁵ parasites were collected and washed two times with PBS 0,01M pH 7.2 and incubated in a lysis solution (100 mM Tris-HCl, 150 mM NaCl, 1 mM EDTA, 1% NP40, 5 □M Trichostatin A, pH 8.8), cells were then centrifuged for 20 min at 10 000 rpm at 4°C. Deacetylase activity in the presence or absence of 200 □M NAD was measured. Results are expressed as relative F355/F460 counts = F355/F460 counts in the presence of NAD - F355/F460 counts in the absence of NAD. This allowed to discriminate between fluorescence due to the action of LmSIR2 to fluorescence due to the presence of compounds which could interfere with the test. As shown in **Figure 2B****,** parasites overexpressing LmSIR2 had more NAD-dependent deacetylase activity than parasites carrying the empty pTEX vector. 5 mM of NAm significantly inhibited the NAD dependent deacetylase activity detected in parasites overexpressing LmSIR2 (**Figure 2B**).

### Example 4

In yeast and *C. elegans*, SIR2 is a limiting component of longevity (reviewed in 3) and NAm is able to accelerate yeast ageing by inhibiting SIR2 *in vivo* (**2**). In the protozoan parasite *L. infantum*, amastigotes carrying extracopies of *LmSIR2* (*LiSIR2*) gene, when maintained under normal axenic culture conditions, showed striking increase in the survival due to an inherent resistance to apoptosis-like death, leading to a longer stationary phase of growth (**11**).

To further examine the possible correlation between the level of SIR2 expression and the sensitivity/resistance to NAm-induced *Leishmania* amastigotes death, NAm was added to cultures of mutant *L. infantum* amastigotes which overexpress LmSIR2 or carrying the empty pTEX plamid as controls.

As shown in **Figures 3** **A and 3B,** adding extra copies of LmSIR2 to amstigotes did not confer significant resistance to NAm-induced death. Thus, even if the NAD-dependent deacetylase activity of LmSIR2 is readily inhibited by NAm and that LmSIR2 play a role in the survival of *Leishmania* amastigotes it should represent only one of the target of NAm mediated cell growth arrest.

The microbicidal mechanism of action of NAm is not currently known. Its activity may come to be understood as that of an indirect antimicrobial that has primarily a prohost effect. Among the reasons to suggest effect is the body of litterature that reports an immunodulatory role for nicotinamide in a wide variety of experimental systems (**8, 7**)**.** Moreover, antioxydant and cryoprotective effect of NAm is well documented (**12**).

Thus, the present invention represents the first report showing the anti-parasitic activity of NAm. Furthermore, although NAm could inhibit the NAD-dependent deacetylase activity of SIR2-like enzymes, its main target in *Leishmania* seems not to be LmSIR2. In fact *Leishmania* possesses two other SIR2 related members whose function and localization are currently unknown. Implication of this protein family in the survival of *Leishmania* parasite has to be investigated. It can be hypothesized that one or all of them are essential for the parasite survival, and that their inhibition leads to the parasite death. Alternatively, other essential physiological functions would be the targets for NAm. The concentration of NAm and Nicotinic acid found to inhibit the intracellular growth of *Leishmania infantum* (IC50 inferior to 2.5 mM) are far higher than those found in whole blood (about 45 □M) but is closer to the plasmatic concentration of nicotinamide achievable (0.7 to 2.3 mM) in patient treated with accelerated radiotherapy for head and Neck cancer (**1**).

In conclusion, nicotinamide is an inexpensive and orally available agent without significant side effects. Since nicotinamide and its derivatives are potentially beneficial components, leishmaniaisis will benefit from therapeutic use of such components, optionally in combination with anti-parasitic drugs.

### References

**1.** Bernier J., M.R.L. Strztford, J Deneckamp, M.F. Dennis, S. Bieri, F. Haven, O Kocagöncü, M. Bolla and A. Rojas. 1998. Pharmacokinetics of nicotinamide in cancer patients treated with accelerated radiotherapy : the experience of the Co-operative group of radiotherapy of the european organization for researc and treatment of cancer. Radiation & Oncology. 48 : 123-133.
**2.** Bitterman K.J., R.M. Anderson, H.Y. Cohen, M. Latorre-Esteves and D.A. Sinclair. 2002. Inhibition of silencing and accelerated aging by nicotinamide, a putative negative regulator of yeast sir2 and human SIRT1. J Biol Chem. 277: 45099-107.
**3.** Imai S., F.B. Joohson, R.A. Marciniak, M. McVey, P.U. Park and L. Garante. 2000. Sir2: an NAD-dependent histone deacetylase that connects chromatin silencing, metabolism, and aging. Cold. Spring. Harb. Symp. Quant. Biol. 65:297-302.
**4.** Kaanders JH., L.A. Pop, H.A. Marres, I. Bruaset, F.J. van den Hoogen, M.A. Merkx and A.J. van der Kogel. 2002. ARCON: experience in 215 patients with advanced head-and-neck cancer. Int J Radiat Oncol Biol Phys. 52: 769-78.
**5.** Landry J., A. Sutton, S.T. Tafrov, R.C. Heller, J. Stebbins, L. Pillus and R. Sternglanz. 2000. The silencing protein SIR2 and its homologs are NAD-dependent protein deacetylases.Proc Natl Acad USA. 97 : 5807-5811.
**6.** Murray M.F and A Srinivasan. 1995. Nicotinamide inhibits HIV-1 in both acute and chronic in vitro infection. Biochem Biophys Res Commun. 210: 954-9.
**7.** Murray M.F. 2003. Nicotinamide: an oral antimicrobial agent with activity against both Mycobacterium tuberculosis and human immunodeficiency virus. Clin Infect Dis. 36: 453-60.
**8.** Otsuka A, T., J. Hanafusa, J. Miyagawa, N. Kono and S. Tarui. 1991. Nicotinamide and 3-aminobenzamide reduce inerferon-gamma-induced class II MHC (HLA-DR and DP) molecule expression on cultured human endothelial cells and fibroblast. Immunopharmacol immunotoxicol. 13 : 263-280. Proc Natl Acad Sci U S A 97: 5807-11.
**9.** Sereno D., P. Holzmuller, I. Mangot, G. Cuny, A. Ouaissi and J.L Lemesre. 2001. Antimonial-mediated DNA fragmentation in Leishmania infantum amastigotes. Antimicrob Agents Chemother. 45:2064-9.
**10.** Sereno, D and J.L. Lemesre. 1997. Axenically cultured amastigote forms as an in vitro model for investigation of antileishmanial agents. Antimicrob Agents Chemother. 41: 972-6.
**11.** Vergnes B., D. Sereno, N. Madjidian-Sereno, J.L. Lemesre and A. Ouaissi. 2002. Cytoplasmic SIR2 homologue overexpression promotes survival of Leishmania parasites by preventing programmed cell death. Gene. 21 : 139-50.
**12.** Yang, J and J.D Adams. 2004. Nicotinamide and its pharmacological properties for clinical therapy. Drug Design Review. 1 : 43-52.

## Claims

1. The use of at least one compound of the following general formula (I): 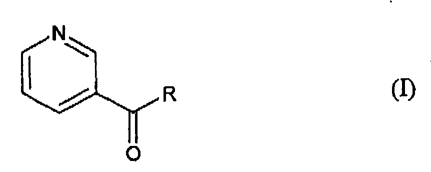 wherein R represents OH or NH₂,
or of the pharmaceutically acceptable salts of said compound, for the manufacture of a medicament intended for the prevention or the treatment of diseases related to *Trypanosomatidae* family, in particular *Trypanosoma* genus or *Leishmania* genus under their respective intracellular or extracellular forms, more particularly of leishmaniosis, and especially for the prevention or the treatment of diseases related to *Trypanosomatidae* family occurring in immunodepressed patients.

2. The use according to claim 1, of a compound of general formula (I), wherein R represents OH, said compound corresponding to niacin (vitamin B3), of the following formula (II):

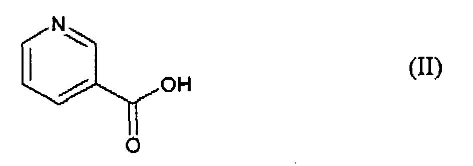

3. The use according to claim 1, of a compound of general formula (I), wherein R represents NH₂, said compound corresponding to nicotinamide, of the following formula (III):

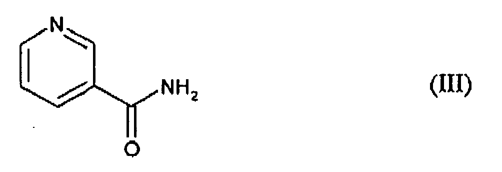

4. The use according to any of claims 1 to 3, wherein the medicament is suitable for an administration by oral, intravenous, topical or intralesional route.

5. The use according to any of claims 1 to 4, wherein the medicament is suitable for an administration of the compound of formula (I) at a unit dose of about 10 mg to about 10 g, in particular of about 1 g to about 6 g.

6. The use according to any of claims 1 to 5, wherein the medicament is suitable for an administration of the compound of formula (I) at a dosage of about 5 mg/m²/day to about 5 g/m²/day, in particular of about 500 mg/m²/day to about 3 g/m²/day.

7. The use according to any of claims 1 to 6, wherein the compound of formula (I) is in association with at least one anti-parasitic compound, such as a compound selected from:
miltefosin, antimonials, amphotericin B, benznidazol, nifurtimox, paromomycin, pentamidin and its derivatives, arsenic derivatives, melarsopol and difluoromethylomithin.

8. A pharmaceutical composition comprising as active substances:
- one compound of the following general formula (I): 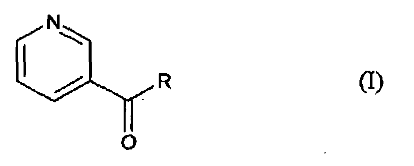 wherein R represents NH₂,
or of the pharmaceutically acceptable salts of said compound, and
- antimonials, - in association with a pharmaceutically acceptable carrier.

9. A pharmaceutical composition according to any of claims 8 suitable for an administration by oral, intravenous, topical or intralesional route.

10. A pharmaceutical composition according to any of claims 8 or 9 , suitable for the administration of the compound of formula (I) at a unit dose of about 10 mg to about 10 g, in particular of about 1 g to about 6 g.

11. A pharmaceutical composition according to any of claims 8 to 10, suitable for the administration of the compound of formula (I) at a dosage of about 5 mg/m²/day to about 5 g/m²/day, in particular of about 500 mg/m²/day to about 3 g/m²/day.

12. Products containing
- compound of the following general formula (I): 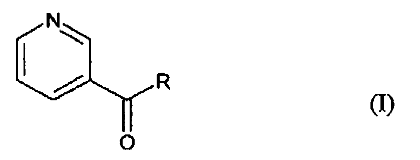 wherein R represents NH₂,
or of the pharmaceutically acceptable salts of said compound, in association with
- antimonials,
as a combined preparation for simultaneous, separate or sequential use in the prevention or the treatment of diseases related to *Trypanosomatidae* family, in particular *Trypanosoma* genus or *Leishmania* genus under their respective intracellular or extracellular form, more particularly of leishmaniosis, and especially for the prevention or the treatment of diseases related to *Trypanosomatidae* family occurring in immunodepressed patients.

## Patentansprüche

1. Verwendung mindestens einer Verbindung mit der folgenden allgemeinen Formel (I): 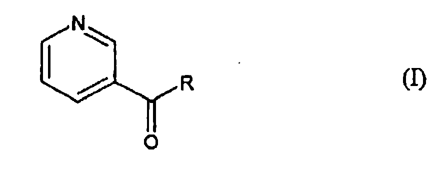 worin R für OH oder NH₂ steht,
oder der pharmazeutisch annehmbaren Salze der Verbindung zur Herstellung eines Medikaments, das zur Vorbeugung oder der Behandlung von Erkrankungen bestimmt ist, die mit der *Trypanosomatidae*-Familie, insbesondere der Gattung *Trypanosoma* oder der Gattung *Leishmania* in ihren jeweiligen intrazellulären oder extrazellären Formen in Zusammenhang stehen, stärker bevorzugt von Leishmaniose und speziell zur Vorbeugung oder der Behandlung von Erkrankungen, die mit der *Trypanosomatidae*-Familie in Zusammenhang stehen, die bei immunsupprimierten Patienten auftreten.

2. Verwendung nach Anspruch 1 einer Verbindung mit der allgemeinen Formel (I), worin R für OH steht, wobei die Verbindung Niacin (Vitamin B3) mit der folgenden Formel (II) entspricht:

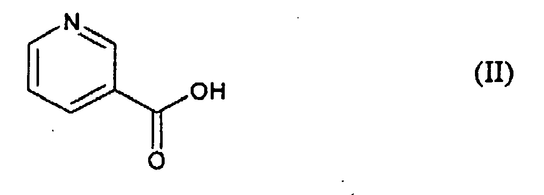

3. Verwendung nach Anspruch 1 einer Verbindung mit der allgemeinen Formel (I), worin R für NH₂ steht, wobei die Verbindung Nicotinamid mit der folgenden Formel (III) entspricht:

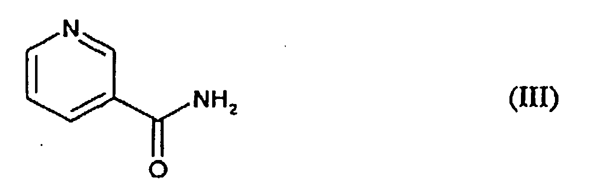

4. Verwendung nach einem der Ansprüche 1 bis 3, wobei das Medikament für eine Verabreichung auf oralem, intravenösem, topischem oder intraläsionalem Weg geeignet ist.

5. Verwendung nach einem der Ansprüche 1 bis 4, wobei das Medikament für eine Verabreichung der Verbindung mit der Formel (I) mit einer Dosiseinheit von etwa 10 mg bis etwa 10 g, insbesondere von etwa 1 g bis etwa 6 g geeignet ist.

6. Verwendung nach einem der Ansprüche 1 bis 5, wobei das Medikament für eine Verabreichung der Verbindung mit der Formel (I) mit einer Dosierung von etwa 5 mg/m²/Tag bis etwa 5 g/m²/Tag, insbesondere von etwa 500 mg/m²/Tag bis etwa 3 g/m²/Tag geeignet ist.

7. Verwendung nach einem der Ansprüche 1 bis 6, wobei die Verbindung mit der Formel (I) mit mindestens einer antiparasitischen Verbindung assoziiert ist, wie etwa einer Verbindung, ausgewählt aus: Miltefosin, antimonhaltigen Mitteln, Amphotericin B, Benznidazol, Nifurtimox, Paromomycin, Pentamidin und deren Derivaten, Arsenderivaten, Melarsopol und Difluormethylornithin.

8. Pharmazeutische Zusammensetzung, die als Wirkstoffe:
- eine Verbindung mit der folgenden allgemeinen Formel (I): 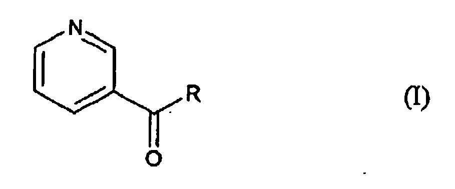 worin R für NH₂ steht,
oder pharmazeutisch annehmbare Salze der Verbindung, und
- antimonhaltige Mittel umfasst, assoziiert mit einem pharmazeutisch annehmbaren Träger.

9. Pharmazeutische Zusammensetzung nach einem der Ansprüche 8, die sich für eine Verabreichung auf oralem, intravenösem, topischem oder intraläsionalem Weg eignet.

10. Pharmazeutische Zusammensetzung nach einem der Ansprüche 8 bis 9, die sich für eine Verabreichung der Verbindung mit der Formel (I) mit einer Dosiseinheit von etwa 10 mg bis etwa 10 g, insbesondere von etwa 1 g bis etwa 6 g eignet.

11. Pharmazeutische Zusammensetzung nach einem der Ansprüche 8 bis 10, die sich für eine Verabreichung der Verbindung mit der Formel (I) mit einer Dosierung von etwa 5 mg/m²/Tag bis etwa 5 g/m²/Tag, insbesondere von etwa 500 mg/m²/Tag bis etwa 3 g/m²/Tag eignet.

12. Produkte, die
- eine Verbindung mit der folgenden allgemeinen Formel (I): 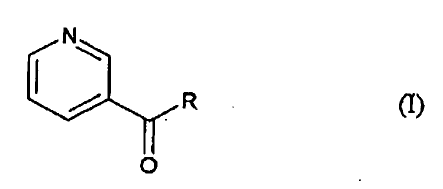 worin R für NH₂ steht,
oder pharmazeutisch annehmbare Salze der Verbindung, und
- antimonhaltige Mittel enthalten,
als Kombinationspräparat zur gleichzeitigen, separaten oder sequenziellen Verwendung bei der Vorbeugung oder der Behandlung von Erkrankungen, die mit der *Trypanosomatidae*-Familie, insbesondere der Gattung *Trypanosoma* oder der Gattung *Leishmania* in ihrer jeweiligen intrazellulären oder extrazellären Form in Zusammenhang stehen, stärker bevorzugt von Leishmaniose und speziell zur Vorbeugung oder der Behandlung von Erkrankungen, die mit der *Trypanosomatidae*-Familie in Zusammenhang stehen, die bei immunsupprimierten Patienten auftreten.

## Revendications

1. Utilisation d'au moins un composé de formule générale (I) suivante: 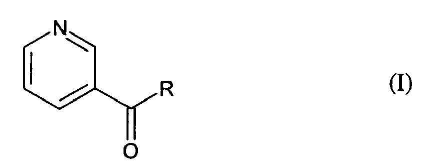 dans laquelle R représente OH ou NH₂,
ou de sels pharmaceutiquement acceptables dudit composé, pour la fabrication d'un médicament destine à la prévention ou au traitement des maladies causées par la famille des *Trypanosomatidae,* en particulier du genre *Trypanosoma* ou *Leishmania,* sous leurs formes respectives intracellulaires ou extracellulaires, plus particulièrement de la leishmaniose, et en particulier pour la prévention ou le traitement des maladies causées par la famille des *Trypanosomatidae* chez les patients immunodéprimés.

2. Utilisation selon la revendication 1, d'un composé de formule générale (I), dans laquelle R représente OH, ledit composé correspondant à la niacine (vitamine B3), de formule générale suivante (II)

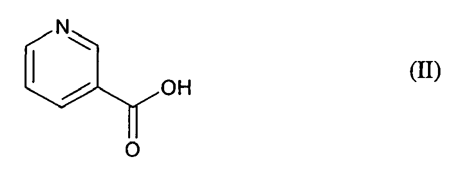

3. Utilisation selon la revendication 1, d'un composé de formule générale (I), dans laquelle R représente NH₂, ledit composé correspondant au nicotinamide, de formule générale suivante (III) :

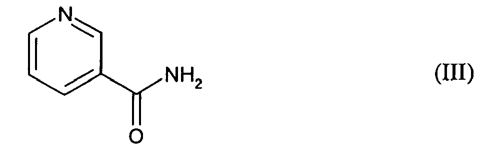

4. Utilisation selon l'une des revendications 1 à 3, dans laquelle le médicament est approprié pour une administration par voie orale, intraveineuse, topique ou intra lésionnelle.

5. Utilisation selon l'une des revendications 1 à 4, dans laquelle le médicament est approprié pour une administration du composé de formule (I) à une dose unitaire d'environ 10 mg à environ 10 g, en particulier d'environ 1 g à environ 6 g.

6. Utilisation selon l'une des revendications 1 à 5, dans laquelle le médicament est approprié pour une administration du composé de formule (I) à une dose d'environ 5 mg/m²/jour à environ 5 g/m²/jour, en particulier d'environ 500 mg/m²/jour à environ 3 g/m²/jour.

7. Utilisation selon l'une des revendications 1 à 6, dans laquelle le composé de formule (I) est en association avec au moins un composé anti-parasitique, tel qu'un composé choisi parmi :
la miltéfosine, l'antimoine, l'amphotéricine B, le benznidazole, le nifurtimox, la paromomycine, la pentamidine et ses dérivés, les dérivés de l'arsenic, le mélarsopol et la difluorométhylornithine.

8. Composition pharmaceutique comprenant comme substance active:
- un composé de formule générale (I) suivante: 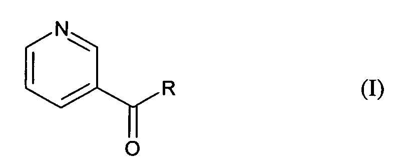 dans laquelle R représente NH₂,
ou de sels pharmaceutiquement acceptables dudit composé, et
- de l'antimoine,
en association avec un transporteur pharmaceutiquement acceptable.

9. Composition pharmaceutique selon la revendication 8, appropriée pour une administration par voie orale, intraveineuse, topique ou intra lésionnelle.

10. Composition pharmaceutique selon la revendication 8 ou 9, appropriée pour une administration du composé de formule (I) à une dose unitaire d'environ 10 mg à environ 10 g, en particulier d'environ 1 g à environ 6 g.

11. Composition pharmaceutique selon l'une des revendications 8 à 10, appropriée pour une administration du composé de formule (I) à une dose d'environ 5 mg/m²/jour à environ 5 g/m²/jour, en particulier d'environ 500 mg/m²/jour à environ 3 g/m²/jour.

12. Produits contenant
- un compound de formule générale (I) suivante: 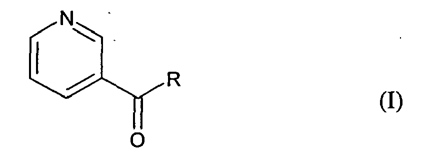 dans laquelle R représente NH₂,
ou de sels pharmaceutiquement acceptables dudit composé, en association avec
- de l'antimoine,
en tant que préparation combinée pour une utilisation simultanée, séparée ou séquentielle pour la prévention ou le traitement des maladies causées par la famille des *Trypanosomatidae,* en particulier du genre *Trypanosoma* ou *Leishmania* sous leur formes respectives intracellulaires ou extracellulaires, plus particulièrement de la leishmaniose, et en particulier pour la prévention ou le traitement des maladies causées par la famille des *Trypanosomatidae* chez les patients immunodéprimés.
